# FASCICULE DE BREVET EUROPEEN

(11) **EP 1 570 968 B1**
(45) Date de publication et mention de la délivrance du brevet: **23.12.2015**
(21) Numéro de dépôt: 05368007.0
(22) Date de dépôt: 28.02.2005
(51) Int. Cl.: A61M 16/00, B29C 41/14, B29C 41/42, B29K 9/06, B29K 21/00, B29K 83/00, B29K 75/00

(54) **Appareil d'assistance respiratoire comprenant une chambre d'expansion**
Beatmungsgerät mit Expansionskammer
Breathing aid apparatus with expansion chamber

(30) Priorité: 01.03.2004 FR 0402093
(43) Date de publication de la demande: 07.09.2005
(73) Titulaire: Koninklijke Philips N.V., 5656 AE Eindhoven (NL)
(72) Inventeur: Delache, Alain, 06300 Nice (FR); Castelo, Véronique, 06300 Nice (FR); Parent, Benoît, 06550 La Roquette sur Siagne (FR)
(74) Mandataire: Verweij, Petronella Daniëlle

(56) Documents cités:
- WO-A-99/22793
- WO-A-99/22794
- DE-A- 10 205 303
- FR-A- 2 663 547
- US-A- 5 590 234
- US-A- 5 647 354
- US-B1- 6 644 311

## Description

La présente invention concerne le domaine des équipements de diminution du bruit à l'intérieur d'un appareil d'assistance respiratoire et plus particulièrement un appareil d'assistance respiratoire comprenant une chambre d'expansion en matière flexible permettant d'éviter la transmission des vibrations de l'organe mécanique, générant le flux d'air à l'intérieur de l'appareil d'assistance respiratoire, à la caisse de l'appareil d'assistance.

Les personnes souffrant de problèmes respiratoires sont assistés dans leur respiration par des dispositifs médicaux en leur délivrant de l'oxygène, en permettant d'ouvrir les voies aériennes ou en assistant la respiration du patient. Les utilisations de ces dispositifs sont aussi bien diurnes que nocturnes.

Ainsi, outre les performances thérapeutiques et inhérentes à ces dispositifs, le confort est associé, par le patient, à la notion de bruit, de vibration, et autres perturbations générées lors du fonctionnement de ces dispositifs.

Deux types de vibrations ou de sons sont générés. Le premier type est dû directement à la circulation du gaz à l'intérieur de l'appareil d'assistance respiratoire. Lorsque l'air circule à travers les cloisons et les conduits d'air, celui-ci crée des turbulences et des vibrations qui se propagent le long des conduits de la caisse dudit dispositif et génèrent ainsi les nuisances sonores et des sensations désagréables, tant pour le patient que pour son entourage. Le deuxième type de vibrations est dû à l'organe mécanique lui-même (piston, turbine ou compresseur) qui génère des vibrations qui lui sont propres et qui se transmettent à la caisse de l'appareil, c'est-à-dire à l'enveloppe extérieure de l'appareil d'assistance respiratoire, entraînant des nuisances sonores et des vibrations désagréables.

Quel que soit l'organe mécanique (piston, turbine, compresseur ou encore concentrateur d'oxygène), le chemin de l'air et l'amortissement des organes mécaniques doivent être optimisés afin de réduire, voir d'éliminer, les nuisances sonores, vibratoires et autres perturbations qui se propagent depuis l'entrée du gaz dans l'appareil d'assistance respiratoire jusqu'au patient.

Afin de lutter contre le second type de nuisances, à savoir les vibrations propres à l'organe mécanique, des mousses sont souvent utilisées pour isoler de la caisse de l'appareil les matériels vibrants, tel que l'organe mécanique ou certains conduits d'air. Dans le document US 6 315 526, cette isolation est effectuée par un coussin d'air. Le conduit dans lequel l'air est envoyé au patient, depuis le compresseur, possède deux trous permettant le passage d'une partie de l'air à l'intérieur de coussins, qui vont être ainsi gonflés et se placer entre l'organe mécanique et les parois internes de la caisse de l'appareil, isolant ainsi l'organe mécanique de cette caisse et créant une sorte d'amortissement mécanique. Cependant, ce dispositif ne permet pas de lutter contre le premier type de nuisances qui résulte de la circulation de l'air dans l'appareil. Tout au plus, il se comporte comme un résonateur de type Helmoltz, qui ne supprime qu'un seul type de fréquences.

Pour lutter contre le premier type de nuisances, différents dispositifs sont parfois mis en place. Il peut s'agir de chicanes ou de cloisons dont le but est de créer des interférences destructrices entre les différentes ondes émises et réfléchies par les cloisons ou à l'intérieur des chicanes. Un autre principe consiste à installer des chambres d'expansion ou silencieux. L'intérêt de ces chambres d'expansion est de permettre une diminution des bruits dus à la circulation de l'air. Le principe consiste en une chambre comportant un orifice d'entrée d'air et un orifice de sortie d'air, de sorte que lorsque l'air entre à l'intérieur de la chambre par l'intermédiaire d'un conduit, celui-ci pénètre dans un volume beaucoup plus grand. Le fait de passer d'un conduit de petite section à ce volume beaucoup plus grand permet une diminution des turbulences et des vibrations, absorbant ainsi le bruit et constituant ainsi un silencieux.

Les chambres d'expansion ont jusqu'ici été peu utilisées dans les appareils d'assistance respiratoire. En effet, pour la plupart de ces appareils, les rotations des turbines des organes mécaniques sont de 5000 à 6000 tours/mn et il est donc nécessaire que les chambres d'expansion aient un volume très élevé. Les quelques chambres d'expansion qui sont utilisées sont en matériau rigide. Si celles-ci permettent une diminution des bruits et vibrations résultant de l'écoulement de l'air, en revanche, leur rigidité entraîne directement la transmission des vibrations émises depuis l'organe mécanique à la caisse de l'appareil d'assistance respiratoire, la chambre d'expansion étant en effet reliée, directement ou indirectement, d'un côté à l'organe mécanique et de l'autre à la caisse de l'appareil d'assistance respiratoire. La solution couramment employée pour lutter contre ces vibrations est de suspendre la chambre d'expansion à l'intérieur de la caisse de l'appareil d'assistance respiratoire, de manière à limiter le contact entre la caisse et la chambre. Cette suspension nécessite l'usage de nombreuses pièces, est difficile à mettre en place et onéreuse. La plupart des appareils essaient de combiner des moyens de résistance à ces nuisances sonores qui couvrent les deux types. Le même type de problème se retrouve pour les appareils utilisant des chicanes ou des cloisons.

Ainsi pour pouvoir diminuer ces différentes vibrations, l'homme du métier a souvent recours à l'emploi de mousses. Celles-ci sont réparties à l'intérieur des conduits ou le long de la paroi de la chambre d'expansion.

Un appareil d'assistance respiratoire comportant une chambre d'expansion est connue de la demande internationale WO 9922793. Ce document montre un appareil d'assistance respiratoire qui est pourvu d'une chambre d'expansion situé dans le passage entre le ventilateur et la partie du dispositif supposé être apposé contre la bouche du patient. Selon WO 9922793, la chambre d'expansion présente des parois rigides. Afin de s'assurer que la nuisance sonore liée à la circulation de l'air dans le dispositif selon cette demande de brevet internationale est réduite, des insertions en mousse on été installées.

L'emploi de ces mousses est particulièrement gênant, tant sur plan de l'hygiène que sur le plan du confort. Celles-ci sont en effet difficiles à nettoyer et retiennent non seulement les poussières mais encore les odeurs. Ainsi, dans la pratique, on a pu constater que les dispositifs médicaux renfermant des mousses absorbent les odeurs du domicile, comme par exemple l'odeur du tabac.

Le but de l'invention est donc de trouver un montage simple permettant une filtration du bruit dû à l'écoulement de l'air et des vibrations mécaniques, tout en étant hygiénique et permettant une utilisation facile.

L'objet de l'invention est un appareil d'assistance respiratoire comprenant au moins une chambre d'expansion, ladite chambre d'expansion comprenant une partie principale et au moins deux orifices, par lesquels ladite chambre d'expansion est reliée aux conduits de circulation de l'air dudit appareil, de manière à ce que l'air rentre dans un volume beaucoup plus grand lorsqu'il passe d'un conduit à l'intérieur de ladite partie principale, permettant ainsi une diminution du bruit lié à la circulation de l'air dans ledit appareil;
caractérisé en ce que ladite chambre esL constituée d'une matière d'une dureté de 15 à 80 shores et d'une épaisseur de 0,075 à 3 mm, conférant ainsi à ladite chambre d'expansion une flexibilité permettant que les vibrations au niveau de l'un des orifices de ladite chambre d'expansion soient diminuées lors de leur transmission à l'autre orifice, par absorption par les parois de ladite chambre d'expansion.

Un autre objet de la présente invention est un appareil d'assistance respiratoire selon l'un des objets de la présente invention, ladite chambre d'expansion étant reliée aux conduits de circulation de l'air entre l'organe mécanique et la caisse de l'appareil d'assistance respiratoire, de sorte que les vibrations dudit organe mécanique ne sont pas transmises à ladite caisse.

Les buts et objets caractéristiques de l'invention apparaîtront plus clairement à la lecture de la description qui suit, faite en référence aux dessins dans lesquels :
La figure 1 représente l'intérieur, vu en perspective, d'un appareil d'assistance respiratoire équipé d'une chambre d'expansion selon la présente invention,
La figure 2 représente une chambre d'expansion telle que celle équipant l'appareil représenté en figure 1 vue en perspective.
La figure 3 représente l'intérieur, vu en perspective, du même appareil d'assistance respiratoire qu'en figure 1 mais équipé d'une chambre d'expansion réalisée différemment,
La figure 4 représente une chambre d'expansion telle que celle équipant l'appareil représenté en figure 3 vue en perspective,
La figure 5 est un diagramme représentant la diminution des vibrations en fonction de la fréquence des vibrations émises par l'organe mécanique de l'appareil d'assistance respiratoire, pour une mode particulier de réalisation.

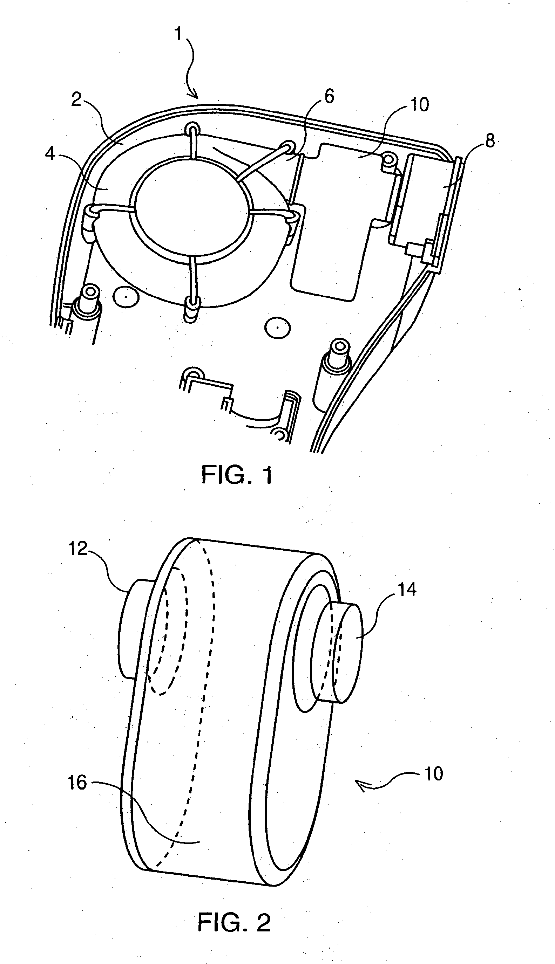

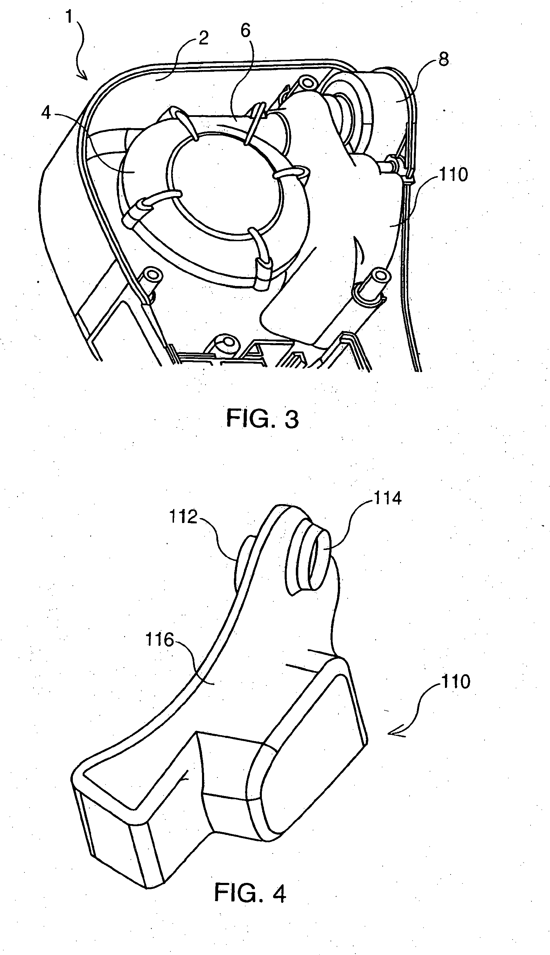

Selon la présente invention et tel que représenté en figure 1 ou 3, la chambre d'expansion 10 ou 110 se place entre la caisse 2 de l'appareil d'assistance respiratoire 1 et l'organe mécanique 4 générant la circulation de l'air à l'intérieur de l'appareil 1 jusqu'à la sortie d'air 8, qui sera connectée au patient, notamment via un masque.

Il est à noter que, si sur les figures 1 eL 3 la chambre d'expansion 10 ou 110 est placée entre le conduit 6 par lequel l'organe mécanique 4 envoie l'air et la sortie d'air 8 de l'appareil 1, il est possible de placer également une chambre d'expansion entre l'entrée d'air de l'appareil d'assistance respiratoire et la prise d'air de l'organe mécanique 4. Ceci permet également d'éviter que les vibrations soient transmises à la caisse 2 via l'entrée d'air de l'appareil d'assistance 1. De la même manière, il est également possible de connecter la chambre 10 ou 110 directement ou indirectement à la caisse 2 ou à l'organe mécanique 4 par l'intermédiaire de conduits d'air. Il est possible d'utiliser plusieurs chambres d'expansion, une à l'entrée de l'appareil d'assistance, une à sa sortie et une ou plusieurs entre les différents éléments à l'intérieur desquels l'air circule.

La chambre d'expansion 10 ou 110 telle que représentée en figure 2 ou 4, comprend une partie principale 16 ou 116 et au moins deux orifices 12 ou 112 et 14 ou 114. Le conduit 6 envoyant l'air depuis l'organe mécanique 4 est relié à l'orifice 12 ou 112 de la chambre d'expansion 10 ou 110. Ainsi lorsque l'air est envoyé par l'organe mécanique 4 à l'intérieur de la partie principale 16 ou 116, il passe d'un conduit 6 de section restreinte à un volume beaucoup plus grand. Cette augmentation de volume permet de diminuer significativement les vibrations dues à la circulation de l'air dans l'appareil. En pratique, plus le volume de la partie principale 16 ou 116 est grand par rapport à la section de l'orifice 12 ou 112, par lequel entre l'air à l'intérieur de la partie principale 16 ou 116, plus ces vibrations seront diminuées. L'air sort ensuite de la chambre d'expansion 10 ou 110, à travers l'autre orifice 14 ou 114 de la chambre d'expansion 10 ou 110, cet autre orifice étant connecté à la sortie d'air 8 de_l'appareil d'assistance respiratoire 1.

La chambre d'expansion 10 ou 110 est constituée d'une matière ayant une flexibilité telle que les vibrations transmises par l'organe mécanique 4 au conduit 6 jusqu'à l'un des orifices 12 ou 112 de la chambre d'expansion 10 ou 110 ne sont pas ou sont faiblement transmis jusqu'à l'autre orifice 14 ou 114 de la chambre d'expansion, ces vibrations étant absorbées par les déformations des parois flexibles de la partie principale 16 ou 116 de la chambre d'expansion. Ainsi les vibrations, propre au fonctionnement de l'organe mécanique 4, ne sont pas transmises à la caisse 2 de l'appareil d'assistance respiratoire 1.

La chambre d'expansion 10 ou 110 permet donc de diminuer les vibrations et nuisances sonores dues tant à la circulation de l'air dans l'appareil d'assistance respiratoire 1 que celles dues aux vibrations de l'organe mécanique 4. L'emploi de mousse n'est ainsi pas nécessaire.

Les flexibilités permettant d'absorber ces vibrations sont obtenues pour avec des matières d'une certaine dureté en fonction de l'épaisseur des parois de la partie principale 16 ou 116. Plus la matière est dure et plus les parois devront être fines pour avoir une flexibilité permettant une diminution notable des vibrations d'un orifice 12 ou 112 à un autre (14 ou 114). En pratique, les meilleurs résultats sont obtenus avec des matières d'une dureté de 15 à 80 shores et une épaisseur de 0,075 à 0,15mm. Lorsque la matière utilisée pour constituer les parois de la chambre d'expansion 10 ou 110, ont une dureté de 30 à 40 shores, l'épaisseur des parois de la chambre d'expansion 10 ou 110 peuvent être comprises entre 0,075 et 0,3 mm. Préférentiellement, la dureté de la matière utilisée pour constituer les parois de la chambre d'expansion 10 ou 110 est comprise entre 30 et 40 shores et l'épaisseur des parois est de 3 mm.

Préférentiellement, la matière utilisée est un élastomère, tel qu'un polymère de silicone, un polymère de styrène-butanediène, ou un polymère de polyuréthane.

Outre des avantages liés à leur propriété de flexibilité, les élastomères permettent d'obtenir la chambre d'expansion 10 ou 110 en une seule pièce et par trempage.

La réalisation de la chambre d'expansion 10 ou 110 en une seule pièce, notamment par moulage ou par trempage, permet d'avoir une pièce adaptable facilement à différents appareils et facilite son entretien, par exemple lors du nettoyage et de la désinfection de ces composants. Les élastomères permettent de réaliser facilement de mode particulier de réalisation en une seule pièce, en permettant le moulage et préférentiellement le trempage.

Préférentiellement la chambre d'expansion 10 ou 110 est donc obtenue par trempage. Pour cela, un moule de la forme de la chambre d'expansion est utilisé. Le procédé comprend les étapes suivantes :
- trempage du moule dans l'élastomère sous forme liquide,
- sortie du moule revêtu d'une pellicule d'élastomère autour du moule,
- solidification de l'élastomère, par séchage ou cuisson, et
- retrait de la chambre 10 ou 110 du moule par l'un des orifices 12 ou 14 (ou 112 ou 114) de la chambre, par déformation des bords de cet orifice.

La dernière étape consistant à extraire le moule de la chambre d'expansion 10 ou 110 est grandement facilité par les matières préférentielles utilisées pour réaliser la chambre d'expansion. Préférentiellement, les élastomères présentent une élasticités de 50 à 60 %, permettant ainsi de déformer, suffisamment l'orifice 12 ou 14 (ou 112 ou 114) pour faire sortir le moule, qui a donc le même volume que la partie principale 16 (ou 116). Ces pourcentages d'élasticité sont préférentiels et l'on peut également envisager une élasticité supérieure à 50 %, par exemple 300 % ou plus, pour faciliter davantage le démoulage.

Un autre objet de la présente invention est donc un appareil d'assistance respiratoire comprenant une chambre 10 ou 110 telle que précédemment décrite, ladite chambre d'expansion étant reliée aux conduits de circulation de l'air entre l'organe mécanique 4 et la caisse 2 de l'appareil d'assistance respiratoire 1 de sorte que les vibrations de l'organe mécanique ne sont pas transmises à la caisse 2.

Préférentiellement la chambre d'expansion 10 ou 110 vient en contact de l'organe mécanique 4 lors du fonctionnement de l'appareil 1 et permet ainsi de dissiper également les vibrations de l'organe de l'organe mécanique 4 par agitation des molécules d'air à l'intérieur de ladite chambre d'expansion et donc dissipation de l'énergie par effet thermique. Il est également possible de positionner la chambre d'expansion 10 ou 110 en contant avec l'organe mécanique 4. Dans ce dernier cas, la chambre d'expansion 10 ou 110 augmente, préférentiellement, son contact avec l'organe mécanique 4 lors du fonctionnement de l'appareil.

Ainsi selon un mode préférentiel de réalisation représenté en figure 3, l'appareil 1 comprend une chambre d'expansion 110, dont la forme lui permet d'épouser en partie la forme de l'organe mécanique 4 et d'améliorer ainsi la dissipation des vibrations par effet thermique. Les matières élastiques utilisées pour réaliser les chambres d'expansion selon la présente invention, sont étirées d'environ 10 % lorsqu'elles sont soumises à la pression d'un appareil respiratoire, la chambre venant ainsi épouser ou épousant davantage la forme de l'organe mécanique 4.

Afin d'optimiser davantage la diminution de transmission des vibrations de l'organe mécanique ou des autres éléments vibrants les différents éléments de l'appareil 1 peuvent être reliés à la caisse 2 par des joints en élastomères.

Il est à noter que, dans le cas d'un organe mécanique 4 équipé d'une turbine, plus la turbine tournera vite, plus les vibrations auront une fréquence élevée et plus les vibrations seront absorbées de manière efficace.

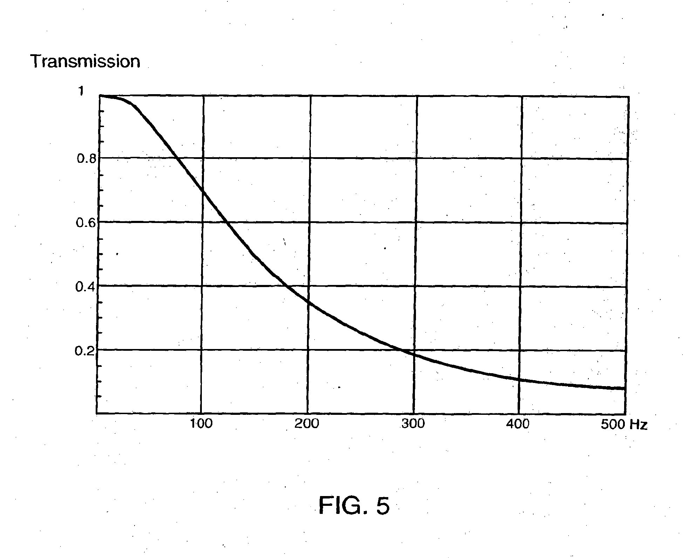

A titre d'exemple des mesures ont été effectuées sur un appareil d'assistance respiratoire équipé d'une chambre d'expansion 10, telle que représentée en figure 2. L'élastomère utilisé pour constituer les parois de la chambre d'expansion est un polymère de silicone d'une dureté de 40 shores. Les parois ont une épaisseur de 0,3 mm. Les mesures sont représentées dans le diagramme, en figure 5, exprimant le rapport de transmission des vibrations en fonction de la fréquence émise par l'organe mécanique. On observe des résultats significatifs dès 100. A 160 Hz la diminution des transmissions des vibrations est déjà de plus de 50 %. A partir de 400 Hz la transmission des vibrations est diminuée de plus de 85 %.

## Revendications

1. Appareil d'assistance respiratoire (1) comprenant au moins une chambre d'expansion (10, 110), ladite chambre d'expansion comprenant une partie principale (16, 116) et au moins deux orifices (12, 112 et 14, 114), par lesquels ladite chambre d'expansion (10, 110) est reliée aux conduits de circulation de l'air dudit appareil (1), de manière à ce que l'air rentre dans un volume beaucoup plus grand lorsqu'il passe d'un conduit (6) à l'intérieur de ladite partie principale, permettant ainsi une diminution du bruit lié à la circulation de l'air dans ledit appareil (1); **caractérisé en ce que** ladite chambre d'expansion (10, 110) est constituée d'une matière d'une dureté de 15 à 80 shores et d'une épaisseur de 0,075 à 3 mm, conférant ainsi à ladite chambre d'expansion (10, 110) une flexibilité permettant que les vibrations au niveau de l'un des orifices (12, 112) de ladite chambre d'expansion (10, 110) soient diminuées lors de leur transmission à l'autre orifice (14, 114), par absorption par les parois de ladite chambre d'expansion (10, 110).

2. Appareil d'assistance respiratoire (1) selon la revendication 1, **caractérisé en ce que** la matière constituant ladite chambre d'expansion (10, 110) a une dureté de 30 à 40 shores et une épaisseur de 0,2 à 0,3 mm.

3. Appareil d'assistance respiratoire (1) selon la revendication 1 ou 2, **caractérisé en ce que** la matière constituant ladite chambre d'expansion (10, 110) est un élastomère tel qu'un polymère de silicone, un polymère de styrène-butadiène ou un polymère de polyuréthane.

4. Appareil d'assistance respiratoire (1) selon la revendication 3, **caractérisé en ce que** l'élastomère constituant ladite chambre d'expansion (10, 110) peut s'étirer de 50% ou plus.

5. Appareil d'assistance respiratoire (1) selon l'une quelconque des revendications précédentes, **caractérisé en ce que** ladite chambre d'expansion (10, 110) est constituée d'une seule pièce.

6. Appareil d'assistance respiratoire (1) selon l'une quelconque des revendications précédentes, **caractérisé en ce que** ladite chambre d'expansion (10, 110) est reliée aux conduits de circulation de l'air entre, l'organe mécanique (4) et la caisse (2) de l'appareil d'assistance respiratoire (1), de sorte que les vibrations dudit organe mécanique ne sont pas transmises à ladite caisse (2).

7. Appareil d'assistance respiratoire (1) selon la revendication 6, **caractérisé en ce que**, lors de la mise en marche de l'appareil (1), ladite chambre d'expansion (10, 110) vient au contact dudit organe mécanique (4) ou augmente son contact avec celui-ci, ladite chambre d'expansion (10, 110) épousant le contour dudit organe mécanique (4) grâce à la flexibilité de la matière la constituant, permettant ainsi de favoriser la dissipation des vibrations dudit organe mécanique (4) par dissipation thermique à l'intérieur de ladite chambre d'expansion (10, 110).

## Patentansprüche

1. Beatmungsgerät (1), das mindestens eine Expansionskammer (10, 110) umfasst, wobei die Expansionskammer einen Hauptteil (16, 116) und mindestens zwei Öffnungen (12, 112 und 14, 114) umfasst, mit denen die Expansionskammer (10, 110) mit den Luftzirkulationsleitungen des Geräts (1) verbunden ist, so dass die Luft in ein sehr viel größeres Volumen eintritt, wenn sie von einer Leitung (6) in das Innere des Hauptteils strömt, was eine Verringerung des mit der Zirkulation der Luft in dem Gerät (1) verbundenen Geräuschs ermöglicht,
**dadurch gekennzeichnet, dass** die Expansionskammer (10, 110) von einem Material mit einer Härte von 15 bis 80 Shore und einer Stärke von 0,075 bis 3 mm gebildet ist, was der Expansionskammer (10, 110) eine Flexibilität verleiht, die erlaubt, die Vibrationen im Bereich einer der Öffnungen (12, 112) der Expansionskammer (10, 110) bei ihrer Übertragung an die andere Öffnung (14, 114) durch Absorption durch die Wände der Expansionskammer (10, 110) zu verringern.

2. Beatmungsgerät (1) nach Anspruch 1, **dadurch gekennzeichnet, dass** das die Expansionskammer (10, 110) bildende Material eine Härte von 30 bis 40 Shore und eine Stärke von 0,2 bis 0,3 mm hat.

3. Beatmungsgerät (1) nach Anspruch 1 oder 2, **dadurch gekennzeichnet, dass** das die Expansionskammer (10, 110) bildende Material ein Elastomer wie ein SilikonPolymer, ein Styren-Butadien-Polymer oder ein Polyurethan-Polymer ist.

4. Beatmungsgerät (1) nach Anspruch 3, **dadurch gekennzeichnet, dass** sich das die Expansionskammer (10, 110) bildende Elastomer um 50 % oder mehr strecken kann.

5. Beatmungsgerät (1) nach einem der vorangehenden Ansprüche, **dadurch gekennzeichnet, dass** die Expansionskammer (10, 110) aus einem einzigen Teil gebildet ist.

6. Beatmungsgerät (1) nach einem der vorangehenden Ansprüche, **dadurch gekennzeichnet, dass** die Expansionskammer (10, 110) mit den Luftzirkulationsleitungen zwischen dem mechanischen Organ (4) und dem Kasten (2) des Beatmungsgeräts (1) derart verbunden ist, dass die Vibrationen des mechanischen Organs nicht auf den Kasten (2) übertragen werden.

7. Beatmungsgerät (1) nach Anspruch 6, **dadurch gekennzeichnet, dass** beim Einschalten des Geräts (1) die Expansionskammer (10, 110) mit dem mechanischen Organ (4) in Kontakt kommt oder seinen Kontakt mit demselben erhöht, wobei die Expansionskammer (10, 110) dank der Flexibilität des sie bildenden Materials an der Kontur des mechanischen Organs (4) anliegt, wodurch erlaubt wird, die Ableitung der Vibrationen des mechanischen Organs (4) durch thermische Ableitung innerhalb der Expansionskammer (10, 110) zu fördern.

## Claims

1. Breathing aid apparatus (1) including at least one expansion chamber (10, 110), said expansion chamber comprising a main section (16, 116) and at least two openings (12, 112 and 14, 114), for connecting said expansion chamber (10, 110) to the air flow ducts of said apparatus (1), such that the air enters into a much larger volume when passing from a duct (6) to within said main section, thus reducing the noise emitted due to the air flowing through said apparatus (1);
**characterised in that** said expansion chamber (10, 110) is made from a material with a hardness of 15 to 80 Shore and a thickness of 0.075 to 3 mm, thereby imparting flexibility to said expansion chamber (10, 110) and thus reducing the vibrations present at one of the openings (12, 112) of said expansion chamber (10, 110) during their transmission to the other opening (14, 114) by absorption by the walls of said expansion chamber (10, 110).

2. Breathing aid apparatus (1) according to claim 1, **characterised in that** the material used to produce said expansion chamber (10, 110) has a hardness of 30 to 40 Shore and a thickness of 0.2 to 0.3 mm.

3. Breathing aid apparatus (1) according to claim 1 or 2, **characterised in that** the material used to produce said expansion chamber (10, 110) is an elastomer such as a silicone polymer, a styrene-butadiene polymer or a polyurethane polymer.

4. Breathing aid apparatus (1) according to claim 3, **characterised in that** the elastomer used to produce said expansion chamber (10, 110) has a stretch capability of 50% or more.

5. Breathing aid apparatus (1) according to any one of the previous claims, **characterised in that** said expansion chamber (10, 110) is made in one piece.

6. Breathing aid apparatus (1) according to any one of the previous claims, **characterised in that** said expansion chamber (10, 110) is connected to the air flow ducts between the mechanical member (4) and the body (2) of the breathing aid apparatus (1), such that the vibrations from said mechanical member are not transmitted to said body (2).

7. Breathing aid apparatus (1) according to claim 6, **characterised in that**, when turning on the apparatus (1), said expansion chamber (10, 110) comes into contact with said mechanical member (4) or increases its contact with the latter, said expansion chamber (10, 110) taking on the shape of the contour of said mechanical member (4) thanks to the flexibility of the material used for its production, thus encouraging the dissipation of vibrations from said mechanical member (4) by thermal dissipation inside said expansion chamber (10, 110).
